# EUROPÄISCHE PATENTANMELDUNG

(11) **EP 1 745 754 A1**
(43) Veröffentlichungstag der Anmeldung: **24.01.2007**
(21) Anmeldenummer: 05015720.5
(22) Anmeldetag: 20.07.2005
(51) Int. Cl.: A61B 17/74, A61B 17/80

(54) **Trochanterplatte**

(71) Anmelder: Prévôt, Bertrand Dr. Med., 22605 Hamburg (DE); Zlowodzki, Michal P. Dr. Med., Minneapolis MN 55403 (US); Bhandari, Mohit M.D., Hamilton ON L9C 6S8 (CA)
(72) Erfinder: Zlowodzki, Michal P. Dr. Med., Minneapolis MN 55403 (US); Bhandari, Mohit M.D., Hamilton Ontario, L9C 6S8 (CA)
(74) Vertreter: Meyer, Ludgerus

(57) **Zusammenfassung**

Die Erfindung betrifft eine Trochanterplatte (1) zur Fixation eines Oberschenkelhalsknochens nach einer Fraktur mittels Knochenschrauben, die anatomisch der Außenseite des proximalen Femur angepasst ist, und die wenigstens eine Bohrung (2, 3, 6) zur Aufnahme einer Zugschraube und wenigstens eine Gewindebohrung (5) zur Aufnahme einer winkelstabilen Schrauben aufweist.

## Beschreibung

Die Erfindung betrifft eine Trochanterplatte zur Fixation eines Oberschenkelhalsknochens nach einer Fraktur gemäß dem Oberbegriff des Anspruchs 1.

Der Oberschenkelhalsknochen ist im menschlichen Körper einer derjenigen Knochen, die, insbesondere bei alten Menschen, bei Stürzen oder Unfällen am häufigsten brechen. Die Bruchstelle befindet sich dabei zwischen dem Oberschenkelkopf und dem proximalen Ende des Femur. Oberschenkelkopf und Femur sind über den Oberschenkelhalsknochen in einem Winkel von etwa 135° (Corpus-Collum-Diaphysenwinkel, auch CCD-Winkel genannt), miteinander verbunden.

Zur Fixation eines gebrochenen Oberschenkelhalsknochens werden in der Regel Knochenschrauben verwendet, die von der Außenseite des Femur durch den Oberschenkelhalsknochen bis in den Oberschenkelkopf geführt werden. Die Längskräfte der Schrauben werden dabei durch den Knochen selbst aufgenommen. Das Einbringen der Schrauben hat mit hoher Präzision zu erfolgen. Ferner sind die Schrauben exakt auszurichten und ihre Köpfe sind an der Oberfläche des Knochens exakt festzulegen.

Gleichwohl ist es bekannt, dass in bis zu 30% der Fälle Nachoperationen erforderlich sind. Ferner existiert eine hohe Sterblichkeit von Patienten mit Oberschenkelhalsbrüchen.

Es ist auch bekannt, die zu verwendenden Knochenschrauben auf der Außenseite des Knochens durch sogenannte Trochanterplatten zu führen, die eine verbesserte Lastverteilung der in die Knochen einzutreibenden Schrauben ermöglichen. Aus der DE 30 17 953 A1 ist eine Endoprothese für den Femurkopf eines menschlichen Hüftgelenkes bekannt. Dort ist ein Schraubenbolzen mindestens teilweise in einer Hülse durch den Schenkelhals hindurchgeführt und mit Hilfe einer Trochanterplatte abgestützt, die ein inneres Kunststofflagerelement enthält, das durch den Zug des Schraubbolzens zusammengedrückt wird. Die Hülse ist im Winkel von etwa 135° etwa parallel zum Oberschenkelhalsknochen ausgerichtet. Ein Nachteil dieser Endoprothesen-Ausbildung liegt darin, dass die Hülse eine relativ große Bohrung im Knochen erfordert und dieser damit weiter geschwächt wird. Ferner ist die Verdrehfestigkeit aufgrund der Verwendung einer einzelnen Schraube nicht sichergestellt.

Aus der DE 35 35 158 ist eine Trochanterplatte bekannt, die eine rechteckige Form aufweist und eine Vielzahl von Bohrungen enthält. Die Trochanterplatte dient in dieser Ausführungsform jedoch nicht der Fixierung eines Oberschenkelhalsknochens, sondern eines Femurbruchs, bei dem ein unmittelbarer Kontakt der Trochanterplatte mit der Bruchstelle besteht.

Zur Fixierung von gebrochenen Oberarmknochen sind aus der DE 101 07 369 A1 und der DE 202 13 100 lmplantatplatten bekannt, die löffelartig ausgebildet sind und mittels einer Vielzahl von Bohrungen an der Außenseite eines Oberarms verschraubt werden können.

In der Chirurgie werden in Verbindung mit Stützplatten zwei Arten von Schrauben verwendet, winkelstabile und nicht winkelstabile Schrauben. Diese können sowohl kortikale als auch Spongiosaschrauben sein. Winkelstabile Schrauben sind Schrauben, die in einem festen Winkel zu einer Stützplatte verlaufen, wobei die Winkelstabilität beispielsweise durch ein in einem bestimmten Winkel verlaufendes Gewinde in der Stützplatte erreicht werden kann, in das der mit einem entsprechenden Gewinde versehene Schraubkopf eingeschraubt wird.

Eine andere Möglichkeit besteht darin, die Schraube per Reibschluss in der Stützplatte winkelstabil festzulegen.

Der Erfindung liegt die Aufgabe zugrunde, eine Trochanterplatte zur Fixation eines Oberschenkelhalsknochens nach einer Fraktur anzugeben, die klein und anatomisch gut verträglich ausgebildet ist, eine präzise Fixierung der Fraktur ermöglicht und einfach und kostengünstig herstellbar ist.

Diese Aufgabe wird durch die im Anspruch 1 angegebene Erfindung gelöst. Vorteilhafte Weiterbildungen der Erfindung sind in Unteransprüchen angegeben.

Gemäß den kennzeichnenden Merkmalen der Erfindung ist die Trochanterplatte anatomisch der Außenseite des proximalen Femur angepasst und enthält wenigstens eine Bohrung zur Aufnahme einer Zugschraube und wenigstens eine Bohrung zur Aufnahme einer winkelstabilen Schraube.

Die anatomische Ausbildung der Trochanterplatte ermöglicht eine präzise Anpassung an die Knochengestalt des Femur und eine optimale Lastverteilung der eingesetzten Schrauben. Mit der verwendeten Zugschraube lässt sich die Fraktur zusammenziehen und positionsfest halten. Die verwendete winkelstabile Schraube verläuft in einem festen Winkel zur Trochanterplatte und unterstützt und stabilisiert die Fraktur, wobei Winkelbelastungen auf die Fläche der Trochanterplatte verteilt werden.

Die erfindungsgemäße Trochanterplatte vereinfacht die Chirurgie bei Oberschenkelhalsfrakturen, verringert die Patientenbelastung und erhöht die Sicherheit zum Neuaufbau der Knochenverbindung. Die Trochanterplatte berührt keine Bruchstelle.

Vorzugsweise enthält die Trochanterplatte zwei oder drei Bohrungen zur Aufnahme von Zugschrauben sowie ein oder zwei Gewindebohrungen zur Aufnahme von winkelstabilen Schrauben. Diese Ausbildung erhöht einerseits die Sicherheit der Schraubbefestigung und andererseits die Stabilität der Knochenverbindung.

Um eine flexible und den jeweiligen anatomischen Verhältnissen anzupassenden Einführung der Zugschrauben zu ermöglichen, ist wenigstens eine der Bohrungen in Langlochform ausgebildet.

Die Trochanterplatte ist vorzugsweise rechteck- bis drachenviereckförmig ausgebildet mit einer Seitenlänge zwischen 2 und 6 cm und einer Materialdicke von 1 - 12 mm. Es wird ein Längen-/Breitenverhältnis < 2,5 bevorzugt. Damit ergibt sich ein relativ kleiner dem Gewebe des Körpers zugewandter Fremdkörper, der gleichwohl einen hohen Funktionsnutzen hat.

Vorzugsweise sind die Bohrungen in den Eckbereichen der Trochanterplatte ausgebildet, um eine optimale Lastverteilung auf der Ebene der Platte zu erzielen. Die Richtung der Bohrungen zur Aufnahme der winkelstabilen Schrauben verläuft vorzugsweise in einem Winkel zur Ebene der Trochanterplatte, der die vollständige Aufnahme der Schrauben in den Oberschenkelhalsknochen erlaubt und der im Wesentlichen dem Corpus-Collum-Diaphysenwinkel (CCD-Winkel) entspricht. Damit wird erreicht, dass eine fixierte winkelfeste Schraubverankerung in Richtung des Oberschenkelhalses erreicht wird.

Vorzugsweise befindet sich eine der winkelstabilen Bohrungen am spitzen unteren Ende der drachenviereckförmig ausgebildeten Trochanterplatte.

Bei ausreichender Dicke der Trochanterplatte kann vorgesehen sein, dass die Bohrungen einen im Durchmesser vergrößerten Teilbereich zur wenigstens teilweisen Aufnahme eines Bereiches des Schraubkopfes aufweisen, um damit das Überstehen des Schraubkopfes von der Trochanterplatte zu verkleinern.

Die Bohrungen können auch eine hinterschnittene Randausnehmung aufweisen, um Schraubenkappen aufzunehmen, die durch Klemmbefestigung die Schraubköpfe abdecken. Dies verbessert die Gleichmäßigkeit der Oberfläche der Trochanterplatte.

Das Material der Trochanterplatte besteht vorzugsweise aus Edelstahl, Keramik, Titan- oder Chrom-Kobalt-Molybdän-Legierungen oder einem physiologisch verträglichen Kunststoffmaterial.

Die Erfindung wird nachstehend anhand eines Ausführungsbeispiels näher erläutert.

Es zeigen:
- Fig. 1: eine Aufsicht auf eine erfindungsgemäße Trochanterplatte,
- Fig. 2: eine Schnittansicht entlang der Linie A-A von Fig. 1,
- Fig. 3: eine Schnittansicht entlang der Linie B-B von Fig. 1,
- Fig. 4: eine Schnittansicht durch eine Bohrung zur Aufnahme einer Knochenschraube 7,
- Fig. 5: eine Schnittansicht einer Bohrung zur Aufnahme einer winkelstabilen Schraube, und
- Fig. 6: eine Darstellung zur Aufnahme einer Abdeckkappe.

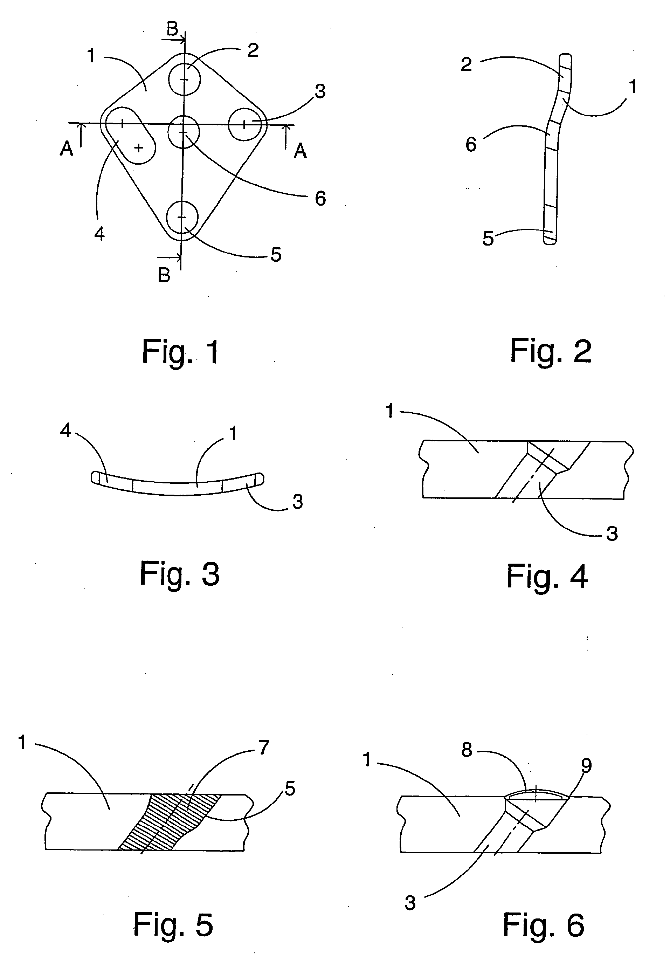

Fig. 1 zeigt eine erfindungsgemäße Trochanterplatte in Aufsicht. Ihre äußere Gestalt ist etwa drachenviereckförmig und ihre größere Längserstreckung verläuft bei ihrem Einsatz in Längsrichtung des Femur. Das Verhältnis zwischen der größeren Längserstreckung und der kleineren Quererstreckung beträgt maximal etwa 2,5. Die Trochanterplatte 1 enthält Standardbohrungen 2, 3 und 6 sowie eine Längsbohrung 4. Diese dienen zur Aufnahme von üblichen Knochenschrauben. Des Weiteren findet sich im unteren spitzen Ende der Trochanterplatte eine Gewindebohrung 5 zur Aufnahme einer winkelstabilen Schraube. Im Ausführungsbeispiel beträgt die Gesamtlänge der Trochanterplatte etwa 3,5 cm und ihre Breite beträgt 2,5 cm.

Fig. 2 zeigt eine Schnittansicht durch die Trochanterplatte entlang der Schnittlinie A-A von Fig. 1. Die Dicke der Platte beträgt etwa 1 - 12 mm, vorzugsweise 3 - 5 mm. Im mittleren Bereich findet sich eine Abbiegung der Platte, die bei ihrer Anwendung unterhalb des Trochanter Major zur Anlage an das Femur kommt.

Fig. 3 zeigt eine Schnittansicht entlang der Linie B-B von Fig. 1. Es ist deutlich gezeigt, dass die Platte eine bogenförmige Gestalt aufweist. Damit ergibt sich eine gute Anpassung an die äußere Form des relevanten Knochenbereichs.

Fig. 4 zeigt eine Schnittansicht durch eine Bohrung 3 zur Aufnahme einer Knochenschraube. Bei sehr dünnen Platten ist keine besondere Ausrichtung der Bohrung 3 vorhanden. Bei einer dickeren Platte 1 kann die Bohrung wie dargestellt im Winkel zur Normalen der Platte ausgerichtet sein, um bei einer winklig eingesetzten Schraube die Passung der Bohrung gegenüber der Schraube zu verbessern. Es kann auch vorgesehen sein, den Schraubkopf in der Trochanterplatte etwas zu versenken, um das Überstehmaß des Schraubkopfes auf der Rückseite der Trochanterplatte zu verkleinern.

Fig. 5 zeigt eine Schrägbohrung 5 mit Gewinde 7, die der Aufnahme einer winkelstabilen Schraube dient. Das Gewinde ist dabei nicht rechtwinklig zur Trochanterplatte angeordnet, sondern in einem Winkel, der es ermöglicht, dass die eingeführte winkelstabile Schraube in dem sogenannten CCD-Winkel zur Trochanterplatte verläuft. Die Gewindeschraube kann eine Imbusaufnahme aufweisen, um ein Vorstehen der Schraube über die Außenseite der Trochanterplatte hinaus möglichst zu vermeiden.

Fig. 6 zeigt eine Möglichkeit der Abdeckung einer Bohrung durch eine Abdeckkappe 8, die hinter einer ringförmig umlaufenden Nase 9 des äußeren Bohrungsrandes geklemmt werden kann.

### Bezugszeichen

- 1: Trochanterplatte
- 2: Bohrung
- 3: Bohrung
- 4: Längsbohrung
- 5: Gewindebohrung
- 6: Bohrung
- 7: Gewinde
- 8: Kappe
- 9: Nase

## Patentansprüche

1. Trochanterplatte (1) zur Fixation eines Oberschenkelhalsknochens nach einer Fraktur mittels Knochenschrauben, **dadurch gekennzeichnet, dass** die Trochanterplatte (1) anatomisch der Außenseite des proximalen Femur angepasst ist, dass die Trochanterplatte (1) wenigstens eine Bohrung (2, 3, 6) zur Aufnahme einer Zugschraube und wenigstens eine winkelstabile Bohrung (5) zur Aufnahme einer winkelstabilen Schraube aufweist.

2. Trochanterplatte nach Anspruch 1, **dadurch gekennzeichnet, dass** die winkelstabile Bohrung eine Gewindebohrung ist.

3. Trochanterplatte nach Anspruch 1, **gekennzeichnet durch** zwei Bohrungen (2, 3) zur Aufnahme von Zugschrauben und zwei Gewindebohrungen (5) zur Aufnahme von winkelstabilen Schrauben.

4. Trochanterplatte nach Anspruch 1, **gekennzeichnet durch** drei Bohrungen zur Aufnahme von winkelstabilen Schrauben und eine Bohrung zur Aufnahme einer Zugschraube.

5. Trochanterplatte nach Anspruch 3 oder 4, **dadurch gekennzeichnet, dass** wenigstens eine der Bohrungen zur Aufnahme von Zugschrauben als Langloch (4) ausgebildet ist.

6. Trochanterplatte nach einem der Ansprüche 1 - 5, **dadurch gekennzeichnet, dass** sie eine im Wesentlichen rechteckige bis drachenviereckfürmige äußere Gestalt mit Seitenlängen zwischen 2 und 6 cm, einer Dicke von 1 - 12 mm und ein Längen-zu-Breiten-Verhältnis von < 2,5 aufweist.

7. Trochanterplatte nach Anspruch 3 und 5 oder 4 und 5, **dadurch gekennzeichnet, dass** die Bohrungen in den Eckbereichen der Trochanterplatte (1) ausgebildet sind.

8. Trochanterplatte nach einem oder mehreren der vorhergehenden Ansprüche, **dadurch gekennzeichnet, dass** die Achsrichtung der Bohrungen zur Aufnahme der winkelstabilen Schrauben in einem Winkel zur Ebene der Trochanterplatte verläuft, der eine vollständige Aufnahme der Schrauben in den Oberschenkelhalsknochen erlaubt.

9. Trochanterplatte nach Anspruch 8, **dadurch gekennzeichnet, dass** der Winkel im Wesentlichen dem Corpus Collum-Diaphysenwinkel (CCD-Winkel) entspricht.

10. Trochanterplatte nach Anspruch 6 und 8, **gekennzeichnet durch** eine am spitzen unteren Ende der drachenviereckförmig ausgebildeten Platte angeordneten winkelstabilen Bohrung.

11. Trochanterplatte nach Anspruch 1, **dadurch gekennzeichnet, dass** die Bohrungen einen äußeren im Durchmesser vergrößerten Teilbereich zur wenigstens teilweisen Aufnahme eines Schraubenkopfes aufweisen.

12. Trochanterplatte nach Anspruch 11, **dadurch gekennzeichnet, dass** die Bohrungen eine hinterschnittene Randausnehmung zur Rastbefestigung einer einen Schraubenkopf abdeckenden Schraubenkappe (8) aufweisen.

13. Trochanterplatte nach einem oder mehreren der vorhergehenden Ansprüche, bestehend aus Edelstahl, Keramik, einer Titan- oder Chrom-Kobalt-MolybdänLegierung oder einem anderen physiologisch verträglichen Metall- oder Kunststoffmaterial.

14. Trochanterplatte nach einem oder mehreren der vorhergehenden Ansprüche, **dadurch gekennzeichnet, dass** jede der Bohrungen für die Aufnahme einer Zugschraube, einer winkelstabilen Schraube oder einer kombinierten winkelstabilen Zugschraube ausgelegt ist.
